# EUROPÄISCHE PATENTANMELDUNG

(11) **EP 3 162 212 A1**
(43) Veröffentlichungstag der Anmeldung: **03.05.2017**
(21) Anmeldenummer: 16002318.0
(22) Anmeldetag: 31.10.2016
(51) Int. Cl.: A22C 13/02

(54) **STOPPERLOSER TUBUS FÜR WURSTDÄRME UND VERFAHREN ZU SEINER HERSTELLUNG**

(30) Priorität: 02.11.2015 DE 102015118745
(71) Anmelder: Reiß, Hilmar, 97342 Obernbreit (DE)
(72) Erfinder: Reiß, Hilmar, 97342 Obernbreit (DE)
(74) Vertreter: Pöhner, Wilfried Anton

(57) **Zusammenfassung**

Tubus zum Transport eines gerafft aufgesteckten Wurstdarms in Lagerungs- oder verarbeitungsfähigem Zustand umfassend einen im Wesentlichen zylindrischen Körper (2), nahe (ca. 1/20 der Länge) beider Enden des Körpers befindliche Vorrichtungen (3) die einen aufgesteckten Wurstdarm gegen ungewolltes Herabfallen sichern, wobei der Tubus inklusive Körper (2) und Sicherung (3) aus einem zusammenhängenden Teil besteht und die beidseitige Sicherung (3) dadurch erfolgt, dass durch gezielte Änderung der Materialeigenschaften, insbesondere das Einbringen von Schwachstellen nahe beider Enden des Körpers (2), dieser so vorbereitet ist, dass mittels gezielter manueller Krafteinwirkungen an jedem Ende jeweils zumindest eine lokale Verdickung herstell- und wieder rückgängig machbarer ist.

## Beschreibung

Die vorliegende Erfindung beschreibt einen Tubus zum Transport eines gerafft aufgesteckten Wurstdarms in lagerungs- oder verarbeitungsfähigem Zustand umfassend einen im Wesentlichen zylindrischen Körper der zumindest auf der mit dem Wurstdarm in Berührung kommenden Außenseite aus lebensmittelechtem Material besteht und nahe der Enden (ca. 1/20 der Länge des Körpers) befindliche Vorrichtungen, die einen aufgesteckten Wurstdarm gegen ungewolltes Herabfallen sichern.

Würste werden hergestellt indem pressfähiges Wurstbrät in einen zur Verarbeitung gewaschenen und geschmeidig gemachten Wurstdarm eingefüllt bzw. gepresst wird. Dies erfolgt aus Gründen der Effizienz mittels sogenannter Wurstfüllmaschinen, die eine längliche Austrittsdüse besitzen, auf die ein zu befüllender Wurstdarm, der ein Natur- oder Kunstdarm sein kann, in gerafftem Zustand aufgesteckt- oder geschoben werden muss, bevor der Befüllvorgang gestartet werden kann.

Früher erfolgte diese Vorbereitung von Hand, d. h. der Metzger oder Fleischer steckte nach und nach den verarbeitungsfertig geschmeidigen Wurstdarm in möglichst sauber und gleichmäßig geraffter Form auf die Düse der Wurstfüllmaschine. Bei den üblichen Produktionsmengen und Wurstdarmlängen von einigen 10 Metern und mehr dauert dieser Vorgang selbst für eine geübte Person mehrere Minuten. Entsprechend lange Stillstandszeiten und entsprechend niedrige Auslastungsgrade der Wurstfüllmaschine ergeben sich dadurch, insbesondere da der eigentliche Füllvorgang vergleichsweise schnell beendet ist und somit ein Wechsel zwischen Wurstdärmen öfter erfolgen muss.

Aus diesem Grund wird schon seit einiger Zeit eine effizientere Methode angewandt, bei ein vom Hersteller fertig vorbereiteter Wurstdarm schon in geraffter Form auf einen Träger, üblicherweise einen festen Schlauch oder einen starren Tubus, gleichmäßig gerafft aufgesteckt und dann für den Transport zur Verarbeitungsstelle gesalzen und getrocknet wird. Vor der Verwendung wird der trockene Darm dann wieder gewässert um ihn geschmeidig und somit verarbeitungsfähig zu machen und wird dann unmittelbar vor Verwendung auf die längliche Düse der Wurstfüllmaschine übertragen. Dies erfolgt indem der Träger, üblicherweise als Hohlzylinder oder Hohlschlauch ausgeführt, an die Düse angesetzt und dann der Wurstdarm unter Erhaltung der gerafften Form als Ganzes vom Träger auf die Düse verschoben wird. Bei einem zur Verarbeitung vorbereiteten geschmeidigen Wurstdarm geht dies sehr leicht, da er sehr gleitfähig ist und zwar in einem solchen Maße, dass dies in der Hektik der Produktion sogar ein Problem darstellen kann. Es kann nämlich leicht vorkommen, dass der Darm in einem unachtsamen Moment ungewollt vom Träger herab fällt. Dies bedeutet in jedem Fall eine gravierende Verzögerung der Verarbeitung und meist kann aus hygienischen Gründen der Wurstdarm nicht mehr verwendet werden.

Dieses Problem des ungewollten Herabfallens eines verarbeitungsfähigen Wurstdarmes vom Trägertubus oder -schlauch wird im Stand der Technik so gelöst, dass ein Stopper in Form eines Rings aus Kunststoff oder Gummi an beiden Enden des Trägers aufgeschoben wird, sodass der geschmeidige Wurstdarm nicht ungewollt herabgleiten kann. Der Nachteil dieser Lösung besteht darin, dass diese Stopper oder Stopperringe kleine Einzelteile darstellen, die oft selbst ungewollt vom Träger rutschen oder nach dem Entfernen aus der Hand und in die Wurstmaschine fallen und dann nur sehr schwierig wieder aufzufinden sind. Selbst wenn der Verlust eines Stoppers bemerkt wird, hat dies auf jeden Fall eine längere Unterbrechung der Produktion zur Folge, bis das Teil wieder aufgefunden ist. Passiert es jedoch unbemerkt, oder zumindest zunächst unbemerkt, wird der Stopper mit verarbeitet und die Wurstware dadurch kontaminiert, was in jedem Fall negative Konsequenzen nach sich zieht, wie zum Beispiel eine aufwendige und teuere Rückrufaktion für ein ganzes Produktionslos.

Weiterhin kommt es in der Wurstproduktion häufig vor, dass mehrere, unter Umständen verschiedene, Wurstdärme auf den selben Trägertubus oder Schlauch aufgesteckt und transportiert werden sollen. Damit diese Wurstdärme nicht aneinander kleben, werden Separatoren eingesetzt, üblicherweise ebenfalls in Form von Kunststoffringen. Obwohl diese Separatoren üblicherweise größer sind als die an den Enden befindlichen Stopper, stellen sie ebenfalls wie diese risikobehaftete Kleinteile dar, die man idealerweise aus dem Produktionsprozess verbannen möchte.

Nach den neuesten Vorschriften müssen diese Stopper und Separatoren nicht mehr nur aus reinem Gummi oder leichtem Kunststoff bestehen, sondern, um leichter auffindbar zu sein, mineralisches (Silikat) oder metallisches Material enthalten. Dadurch sind sie zwar bei Verlust im Prinzip leichter mittels spezieller Maschinen wieder auffindbar, aber das Risiko, dass von einem nicht wieder aufgefundenen Kleinteil ausgeht, erhöht sich dadurch, denn diese mineralischen oder metallischen Markierungsmaterialien sind weniger lebensmittelecht als die bisher verwendeten reinen Kunststoffe.

Die vorliegende Erfindung stellt sich vor diesem Hintergrund die Aufgabe einen Träger für einen Wurstdarm in Form eines Tubus zu entwickeln, der nur aus einem zusammenhängenden Teil besteht und bei dem verarbeitungsfähige, in geraffter Form aufgesteckte Wurstdärme ohne Verwendung eines zusätzlichen Stopper gegen Herabfallen sicherbar sind.

Gelöst wird diese Aufgabe durch einen Tubus mit den in Anspruch 1 angegebenen Merkmalen. Als gezielte Materialveränderung eignen sich insbesondere mechanisch, chemisch oder mittels Bestrahlung durch elektromagnetische oder Partikelstrahlung in ein ansonsten weitgehend homogenes Tubusmaterial eingebrachte Schwachstellen. Denkbar ist aber auch das Aufbringen zusätzlichen Materials oder einen Kompositaufbau aus eigentlichem Trägermaferial, zum Beispiel einem lebensmittelechten Kunstoff, und darin eingebettem Material, wie Lamellen, welche die plastische Verformbarkeit verbessern und/oder (Soll-)Bruch oder Biegestellen definieren. Wichtig ist hierbei, dass mit möglichst geringen Handkräften dauerhafte, von Teil zu Teil reproduzierbar ausgeformte Verdickungen, also Stellen mit vergrößertem Querschnitt, erzeugt werden können, die idealerweise gegenüber ungezielter Krafteinwirkung, z.B. beim Transport unvermeidbar auftretende Druck- und/oder Zugkräfte, möglichst robust sind.

Der Verwendungsablauf des erfindungsgemäßen stopperlosen Tubus ist dann wie folgt:
Angenommen ein frisch produzierter Wurstdarm, der in gerafftem Zustand auf einer Stange aufgesteckt vorliegt, soll auf einen Tubus nach Anspruch 1 übertragen werden. Dann wird zunächst mittels einer gezielten manueller Krafteinwirkung seitens des Anwenders auf eine der entsprechend vorbereiteten Stellen nahe eines ersten Endes des erfindungsgemäßen Tubus eine Verdickung hergestellt, wodurch der Wurstdarm nicht mehr über diese Stelle gleiten kann. Dann wird der Tubus mit dem noch offenen zweiten Ende an ein offenes Ende der den Wurstdarm tragende Stange gehalten, sodass Stange und Tubus in einer Flucht stehen, dann der geraffte Wurstdarm als Ganzes auf den Tubus verschoben, und schließlich durch eine weitere manuelle Krafteinwirkung auf das zweite Ende des Tubus eine zweite Verdickung hergestellt, sodass nun der Wurstdarm beidseitig gegen ungewolltes Herabfallen gesichert ist.

Der Wurstdarm kann nun auf dem erfindungsgemäßen Tubus als Träger gesalzen, getrocknet und so für den Transport oder für die Lagerung vorbereitet werden. Vor der Verarbeitung wird dann der trockene Wurstdarm wieder gewässert um ihn geschmeidig zu machen.
Um ihn auf die Düse einer Wurstfüllmaschine zu übertragen, setzt man nun den Tubus mit aufgebrachtem Wurstdarm mit dem ersten Ende fluchtend, d.h. koaxial, an der Düse der Wurstfüllmaschine an, hierfür bietet es sich an, wenn der Tubus ein Hohlzylinder ist, der einen Innendurchmesser hat, der dem Außendurchmesser der Wurstfülldüse entspricht, denn dadurch lässt sich eine feste Verbindung herstellen und das erste Ende muss während des weiteren Übertragungsvorgangs nicht von Hand unterstützt werden. Ist die Verbindung hergestellt, macht man mit Hilfe einer manuellen, meist durch eine der zur Herstellung nötigen entgegengesetzten Krafteinwirkung die Verdickung am ersten Ende des Tubus wieder rückgängig, und schiebt dann den geschmeidigen Wurstdarm als Ganzes vom Tubus auf die Düse. Der Tubus hat nun seine Aufgabe als Träger erfüllt und kann entweder entsorgt oder eventuell auch gesäubert und wiederverwendet werden.

Der wesentliche Vorteil dieses Tubus zum Transport von Wurstdärmen ist, dass er nur aus einem einzigen Teil besteht und für die Sicherung gegen Herabfallen keine zusätzlichen Kleinteile in Form von Stoppern benötigt. Dies hat viele vorteilhafte Konsequenzen, vor allem in Form eines reibungsloseren Produktionsablaufs, da keine Kleinteile mehr in die Wurstmaschine fallen und entweder, falls bemerkt, die Produktion unterbrechen oder, falls unbemerkt, die Qualität des Endproduktes herabsetzen können. Es muss keine aufwändige Suche nach einem verloren gegangenen Stopper mehr durchgeführt werden, und ebenso ist kein, unter Umständen teures, Spürgerät zur Suche dieser Kleinteile im Wurstbrät oder in der Wurstmaschine nötig.

Der Verzicht auf Stopper hat auch noch den weiteren Vorteil, dass bei jedem eingesetzten Tubus ein kleiner Geldbetrag gespart wird, da die Stopper zwar sehr simple und billige Kleinteile sind, aber durch die in der Wurstproduktion eingesetzte hohe Zahl solcher Vorrichtungen, trotzdem nicht vernachlässigbare Summen zusammenkommen.

Neben dieser finanziellen Einsparung gibt es auch noch den Vorteil einer einfacheren Lagerhaltung, denn bei den nach dem Stand der Technik eingesetzten Tuben mit Stoppern müssen immer beide Teile in gleicher Menge bestellt und vorrätig gehalten werden bzw. im Verhältnis 2 : 1 Stopper zu Tuben. Bei den einteiligen Tuben gemäß der hier offenbarten Erfindung ist dies nicht mehr der Fall und es muss nur noch ein einziges Teil bereitgehalten werden.

Des Weiteren wird die eigentliche Funktion der Stopper, nämlich den verarbeitungsfähigen Wurstdarm gegen ungewolltes Herabfallen zu sichern, durch die erfindungsgemäßen Tuben ebenfalls besser erfüllt. Denn die im Stand der Technik bekannten Stopper können bei übermäßiger Belastung d. h. bei übermäßigem Schräghalten des mit dem gerafften Wurstdarm versehenen Trägertubus auch von selbst nachgeben und so den Wurstdarm herabgleiten lassen, insbesonders wenn durch Verwechslung oder aus anderen Gründen nicht zueinanderpassende Teile kombiniert wurden. Bei der hier beschriebenen Methode, die Sicherung durch eine manuell herstellbare Verdickung der Enden des Trägertubus selbst zu erreichen, ist, bei der Wahl eines geeigneten Tubusmaterials und geeignet ausgestalteter Materialveränderung, eine höhere Widerstandsfähigkeit gegen axiale Kräfte, wie sie von einem feuchten, und damit schweren, Wurstdarm ausgehen,gegeben.

Darüber beschleunigt der erfindungsgemäße Tubus den Produktionsablauf, da bei einem Tubus mit Stopper immer mindestens zwei Handgriffe nötig sind um einmal den Stopper aufzunehmen und dann auf den Tubus aufzustecken, wo hingegen bei einem erfindungsgemäßen Tubus nur ein Handgriff nötig ist.

Ebenso lässt sich die hier offenbarte Methode der Sicherung eines Wurstdarms gegen Herabgleiten durch Herstellung einer Verdickung des Tubuskörpers genauso gut einsetzen, um eine Separierung verschiedener Wurstdärme gegeneinander zu erreichen und somit ist der ,stopperlose Tubus' gleichfalls als ein ,separatorloser Tubus' ausführbar. Entsprechend kann man mit der hier offenbarten Methode das Idealziel erreichen, alle potentiell problematischen Kleinteile aus der Wurstproduktion zu eliminieren.

Im Folgenden sollen vorteilhafte Weiterbildungen der Erfindung, die einzeln oder in Kombination realisierbar sind, vorgestellt werden.

Die Materialveränderung zur Herstellung der Verdickung an den Enden und damit zur Sicherung des Wurstdarms gegen Herabfallen erfolgt vorteilhafterweise dadurch, dass bei einem Tubus, dessen Körper ein Kunststoffhohlzylinder ist, nahe der Enden, also dort wo die Sicherung erfolgen soll, eine gezielte Schwächung des Zylindermaterials vorgenommen wird. Dies kann vorteilhafterweise insbesondere erreicht werden durch Einbringen von Rillen oder Vertiefungen, entweder auf die Innen- oder Außenseite oder auf beiden, oder auch von komplett durch das Hüllenmaterial hindurchreichenden Schlitzen. Bei geeigneter Ausformung dieser Rillen oder Schlitze lässt sich dann mit Hilfe manueller Krafteinwirkung eine von Teil zu Teil reproduzierbar geformte und auch von Hand wieder rückgängig machbare lokale Verdickung, z.B. in Form eines mehr oder minder rotationssymmetrischen Aufpilzung oder Aufwölbung, des Tubusmaterials nahe des Endes erreichen, die zur Sicherung eines Wurstdarms gegen ungewolltes Herabfallen geeignet ist.

Als besonders vorteilhaft hat sich eine Form der Materialschwächung bewährt, bei der nahe der Enden d. h. ca. 1/20 der gesamten Tubuslänge vom Ende entfernt, eine Reihe gleichlanger, parallel zueinander verlaufender Schlitze eingebracht wird, die einen von Null verschiedenen Winkel mit der Axialrichtung bilden. Dies hat den Vorteil, dass der Anwender mittels einer einzigen schraubenartigen Bewegung ein gleichmäßiges Aufpilzen bzw. Aufwölben der durch die Schlitzung geschaffenen Lamellen erreichen kann.

Um eine möglichst symmetrische Aufwölbung zu erhalten, empfiehlt es sich, benachbarte Schlitze immer im gleichen Abstand zueinander einzubringen. Auch sollte die Zahl der Schlitze nicht zu klein, bzw. ihr Abstand nicht zu groß gewählt werden. In der Praxis hat sich z.B. bei einem 15mm durchmessenden Tubus ein Schlitzabstand von ca. 3mm (dies entspricht 15 bis 16 Schlitzen) bewährt.

Der Grad der erreichbaren Verdickung, d. h. in welchem Maße der Außendurchmesser des Zylinders an der so lamellierten Stelle effektiv vergrößert werden kann, hängt davon ab, wie lang die Schlitze sind, und wie weit die Schraubung vorgenommen wird. In grober Näherung kann man sagen, dass bei einer annähernd maximalen Verformung die Änderung des Außendurchmessers ungefähr der halben Schlitzlänge entspricht. Eine maximale Verformung liegt vor, wenn sich die ungeschlitzten Außen- und Mittelteile des Tubus berühren.

In der praktischen Verwendung ist es aus mindestens zwei Gründen sinnvoll, eine maximale Verformung herzustellen. Zum einen gibt eine größere Verdickung einen besseren Schutz gegen ein Herabrutschen des Wurstdarms (durch die größere Dicke selbst sowie aufgrund der steileren Flanke) zum anderen bleibt eine maximale Verformung auch länger erhalten. Da der Tubus aus, in gewissen Grenzen, flexiblem Material besteht, würde eine kleine Schraubung nur eine elastische Verformung bewirken. Wird die Schraubung jedoch soweit als möglich fortgesetzt, entsteht eine plastische Verformung und damit die gewünschte dauerhafte Sicherung.

Die zur Herstellung dieser Verdickung nötige Schraubbewegung muss dabei so gerichtet sein, dass man mit der Hand in die Richtung dreht, die bei Draufsicht auf das Ende des Tubus durch die leicht schräg verlaufenden Schlitze vorgegeben ist. Verlaufen die Schlitze z. B., in Seitenansicht von der Mitte des Tubus zum Ende gesehen, von links nach rechts, so geben diese Schlitze bei Draufsicht auf dieses Ende einen im Urzeigersinn gerichteten Drehsinn vor. D.h. um ein Aufpilzen bzw. Wölben zu erreichen, muss man mit der Hand das Ende des Tubus im Uhrzeigersinn gegen den Mittelteil verdrehen. Nach einer anfänglichen Rotation kann man den Verformungsprozess unterstützen und beschleunigen, indem man zusätzlich zur Drehung Ende und Mittelteil axial leicht gegeneinander schiebt, d.h. effektiv eine Schraubbewegung ausführt. Verlaufen die Schlitze hingegen von rechts nach links so hat die Drehung bzw. Schraubung gegen Uhrzeigersinn zu erfolgen.

Ist eine plastische Verformung einmal erreicht, hält sie im Prinzip beliebig lange. Sie kann aber von Hand wieder rückgängig gemacht werden, indem eine Schraubung in der entgegengesetzten Richtung zur für die Herstellung nötigen angewandt wird. D. h., um wieder das Beispiel der von links nach rechts verlaufenden Schlitze zu bemühen, dass die Aufpilzung bzw. Aufwölbung der Lamellen durch eine Drehung gegen den Uhrzeigersinn und ein Ziehen des Endes des Tubus vom Mittelteil weg wieder rückgängig gemacht werden kann.

Ein Vorteil dieser Methode besteht darin, dass tatsächlich nur eine-Schraubbewegung die gewollte Verformung erreicht, also eine reine Zugkraft nicht ausreichend ist. Dies stellt einen willkommenen Schutz gegen ein ungewolltes Rückgängigmachen der Verformung, z.B. durch während des Transports auftretende axiale Kräfte dar.

In einer vorteilhaften Ausführung der hier beschriebenen Erfindung wird weiterhin darauf geachtet, dass nicht nur die Schlitze an einem Ende untereinander, sondern auch Schlitze verschiedener Enden zueinander parallel sind. Dies hat den positiven Effekt, dass ein Anwender so zum Herstellen bzw. Rückgängigmachen einer Verdickung an beiden Enden eine gleichgerichtete Schraubung vornehmen kann, was es Anwendern erleichtert, sich eine Arbeitsroutine anzueignen.

Um den in der hier offenbarten Erfindung vorgestellte Methode erfolgreich anwenden zu können, muss der Körper des Tubus aus einem einigermaßen nachgiebigen und flexiblen aber dennoch festen Material hergestellt sein. In vorteilhafter Weise ist dies ein lebensmittelechter Kunststoff.

Natürlich steht es frei, außer den Schwachstellen an den Enden auch noch weitere Schwächungen des Tubusmaterials vorzunehmen. Dort kann dann in gleicher Weise durch eine entsprechend gerichtete Schraubung eine Verdickung oder Aufpilzung des Tubus erreicht werden, die z. B. zur Separierung verschiedener auf dem Tubus zu transportierender Wurstdärme verwendbar sind.

Da in der Praxis ein einmal von einem Ende her aufgeschobener Wurstdarm nur über das andere Ende unter Erhaltung einer sauber und gleichmäßig gerafften Form als Ganzes verschoben werden kann, lehrt eine weitere vorteilhafte Ausführungsform, den erfindungsgemäßen Tubus mit Markierungen zu versehen, z.B. Farbmarkierungen, die eine Eindeutige Identifizierung der Enden ermöglichen.

Im Folgenden sollen anhand der Figuren bevorzugte Ausführungsbeispiele der vorliegenden Erfindung näher erläutert werden. Dies dient nur der Illustration und soll die vorliegende Erfindung in keiner Weise einschränken.

Es zeigen
- Figur 1:: Eine Draufsicht auf eine bevorzugte Ausführung des erfindungsgemäßen Tubus.
- Figur 2:: Einen vergrößerten Ausschnitt eines Endes des Tubus nach Figur 1.

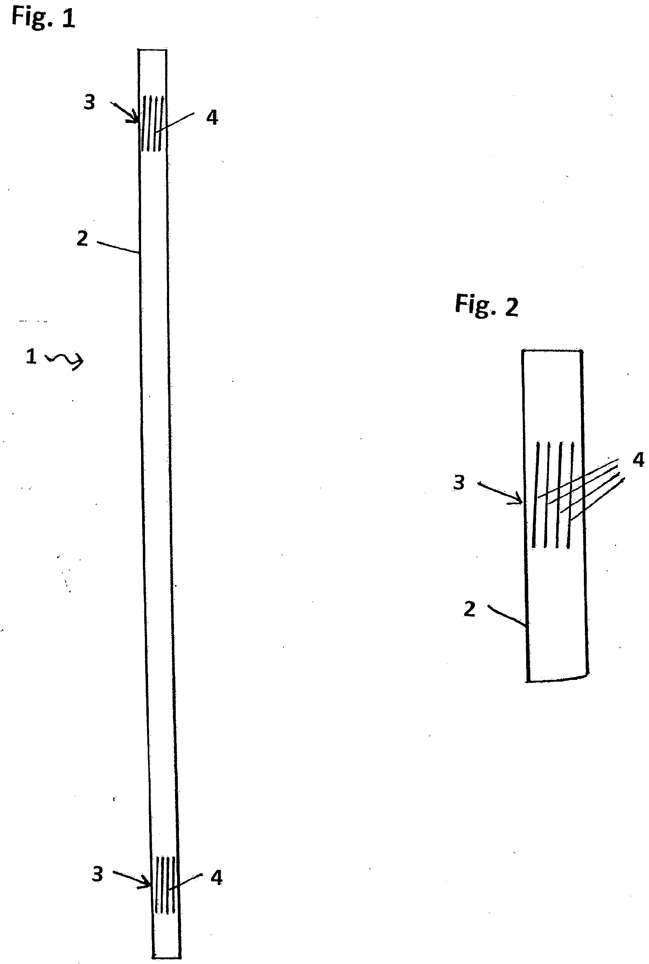

Figur 1 zeigt in Draufsicht eine bevorzugte Ausführung des erfindungsgemäßen stopperlosen Tubus 1, bei dem die zur Herstellung der Verdickung nötige Schwächung des Materials 3 in Form von gleichlangen, parallel verlaufenden Schlitzen 4, hier schematisch als schräge Linien angedeutet, vorgenommen wurde, die außerdem mit der Axialrichtung einen spitzen Winkel von ca. 10 Grad bilden und bei denen benachbarte Schlitze 4 immer den gleichen Abstand haben. Der Körper 2 des Tubus 1 ist idealerweise ein Hohlzylinder aus einem flexiblen, lebensmittelechten Kunststoff.

Die Schlitze verlaufen in dem hier abgebildeten Ausführungsbeispiel an beiden Enden in Draufsicht von unten nach oben gesehen von links nach rechts wodurch für beide Enden eine gleichgerichtete Schraubung zur Erzeugen einer Aufpilzung bzw. -wölbung nötig ist, welche, von oben gesehen, im Uhrzeigersinn zu erfolgen hat. Dies ist vorteilhaft, da ein Anwender so zum Herstellen einer Verdickung an beiden Enden eine gleichartige Schraubung vornehmen kann. Selbiges gilt auch für das Rückgängigmachen der Aufwölbungen. Dies hat weiterhin den Vorteil, dass der Tubus drehsymetrisch ist, wodurch es nicht relevant ist von welchem Ende her der geraffte Wurstdarm aufgeschoben wird. Ist dies aber einmal geschehen, so ergibt sich aus technischen Gründen, dass der Darm nur in die gleiche Richtung (also über das andere Ende) wieder vom Tubus herunter geschoben werden kann, da in der Praxis nur so ein verarbeitungsfähige Wurstdarm unter Erhaltung seiner gleichförmig gerafften Form als Ganzes verschoben werden kann. Somit empfiehlt es sich, wenn der auf dem Tubus durch entsprechende Markierungen die Enden eindeutig identifizierbar gemacht werden, damit das Aufschieben bzw. Herunterschieben des Wurstdarmes immer nur von einem Ende herein und über das andere Ende herunter erfolgt.

Figur 2 zeigt einen vergrößerten Ausschnitt eines Endes der bevorzugten Ausführung des erfindungsgemäßen Tubus 1 aus Figur 1. Zu sehen ist in Draufsicht eine schematische Darstellung des Tubus 1 der in Form eines Plastikhohlzylinders 2 gegeben ist, und den als schräge Linien angedeuteten, parallel verlaufenden Schlitzen 4, die nahe des Endes in das Füllmaterial des Tubus eingebracht sind und die mit der Axialrichtung einen spitzen Winkel von ca. 10 Grad bilden.

Ein Beispiel einer in der Praxis häufig anwendbaren Ausführung ist wie angedeutet 500 mm lang, die parallelen Schlitze 4 beginnen ca. 25 mm von beiden Enden entfernt, sind ca. 30 mm lang, haben einen Abstand von ca. 3 mm und bilden einen Winkel von ca. 10 Grad mit der Axialrichtung. Der Außendurchmesser des Tubus 1 ist hier ungefähr 15 mm. Andere Ausführungsgrößen sind natürlich genauso denkbar, jedoch sollte in jedem Fall darauf geachtet werden, dass der Innendurchmesser des Hohlzylinders dem Außendurchmesser der Düse der Wurstfüllmaschine entspricht, um ein einfaches Ansetzen zu ermöglichen. Weiterhin sind allzu große Wandstärken des Hohlzylinders 2 der Umsetzung der Erfindung abträglich, da die von Hand aufzubringenden Kräfte dadurch erhöht würden und zusätzlich hierzu stiegen auch das Gewicht des Tubus 1 und damit die Herstellungskosten.

### Bezugszeichenliste

- 1: Tubus
- 2: Tubuskörper (Hohlzylinder)
- 3: Sicherungsstelle mit Materialschwächung
- 4: parallele, gewinkelte Schlitze

## Patentansprüche

1. Tubus (1) zum Transport eines gerafft aufgesteckten Wurstdarms in Lagerungs- oder verarbeitungsfähigem Zustand umfassend
- einen im Wesentlichen zylindrischen Körper (2)
- nahe (ca. 1/20 der Länge) beider Enden des Körpers befindliche Vorrichtungen (3) die einen aufgesteckten Wurstdarm gegen ungewolltes Herabfallen sichern
**dadurch gekennzeichnet, dass**
- der Tubus (1) inklusive Körper (2) und Sicherung (3) einstückig ist
- die beidseitige Sicherung (3) durch gezielte Änderung der Materialeigenschaften, insbesondere durch das Einbringen von Schwachstellen nahe beider Enden des Körpers (2), auf eine solche Weise hergestellt ist, dass mittels gezielter manueller Krafteinwirkungen an jedem Ende jeweils zumindest eine lokale Verdickung herstell- und wieder rückgängig machbar ist.

2. Tubus nach Anspruch 1, **dadurch gekennzeichnet**, **dass**der Körper (2) ein Hohlzylinder ist und die Änderung der Materialeigenschaften in Form von auf der Außen- oder Innenseite eingebrachten Rillen oder vollständig durch das Material hindurchgehenden Schlitzen gegeben ist.

3. Tubus nach Anspruch 2, **dadurch gekennzeichnet, dass** die gezielte Schwächung des Materials des Körpers (2) nahe beider Enden in Form von gleichlangen (ca. 1/15 der Länge des Körpers), parallel verlaufenden Schlitzen (4), die zur Axialrichtung einen Winkel bilden, gegeben ist.

4. Tubus nach Anspruch 3, **dadurch gekennzeichnet**, **dass**benachbarte Schlitze (4) alle den gleichen Abstand zueinander sowie die Anfangspunkte aller Schlitze (4) den gleichen Abstand zum Ende des Körpers (2) besitzen.

5. Tubus nach Anspruch 4, **dadurch gekennzeichnet**, **dass**die Schlitze beider Enden parallel verlaufen.

6. Tubus nach einem der vorhergehenden Ansprüche, **dadurch gekennzeichnet**, **dass**der Körper (2) aus einem lebensmittelechten Kunststoff besteht.

7. Tubus nach einem der vorhergehenden Ansprüche, **dadurch gekennzeichnet**, **dass**zusätzlich zur Sicherung (3) an weiteren Stellen des Tubus, insbesondere zwischen den Sicherungen (3), weitere zur manuellen Herstellung einer Verdickung geeignete Schwachstellen vorhanden sind.

8. Tubus nach einem der vorhergehenden Ansprüche, **dadurch gekennzeichnet, dass** Markierungen vorhanden sind, die die Enden eindeutig identifizieren.

9. Verfahren zur Verwendung eines stopperlosen Tubus umfassend
- einen an einem Herstellungsort auf einer Stange in verarbeitungsfähigem Zustand gerafft aufgesteckten Wurstdarm
- einen am gleichen Ort befindlichen Tubus nach einem der Ansprüche 1 bis 8
- eine an einem Verarbeitungsort befindliche Wurstfüllmaschine mit länglicher Düse, welche eine zur Aufnahme des Wurstdarmes nötige Länge besitzt,
**dadurch gekennzeichnet, dass**
- von Hand an einem ersten Ende des Tubus eine als Sicherung gegen Herabfallen des Wurstdarms dienende Verdickung hergestellt und der Tubus mit seinem zweiten, noch offenen, Ende an ein offenes Ende der den Wurstdarm tragenden Stange fluchtend angesetzt wird
- der Wurstdarm als Ganzes auf den Tubus aufschoben und anschließend von Hand am zweiten Ende des Tubus ebenfalls eine Verdickung hergestellt wird
- der Tubus von der Stange entfernt und der Wurstdarm, insbesondere durch Salzen und/oder Trocknen, für einen Transport oder eine Lagerung vorbereitet wird
- der Tubus mitsamt Wurstdarm an den Verarbeitungsort verbracht und dort der Wurstdarm, insbesondere durch Wässern, für die Verarbeitung vorbereitet und gleitfähig gemacht wird
- an einem Ende des Tubus die Verdickung von Hand rückgängig gemacht, der Tubus mit diesem Ende fluchtend an die Düse der Wurstfüllmaschine angesetzt und der Wurstdarm als Ganzes auf die Düse geschoben wird.
